## Europäisches Patentamt

## European Patent Office

## Office européen des brevets

(11) Veröffentlichungsnummer: **0 062 219**
**A2**

## (12) EUROPÄISCHE PATENTANMELDUNG

(21) Anmeldenummer: **82102362.9**

(22) Anmeldetag: **22.03.82**

(51) Int. Cl.³: **A 61 B 6/02**

(30) Priorität: **02.04.81 DE 3113368**

(43) Veröffentlichungstag der Anmeldung: **13.10.82**
**Patentblatt 82/41**

(84) Benannte Vertragsstaaten: **CH FR GB IT LI**

(71) Anmelder: **SIEMENS AKTIENGESELLSCHAFT Berlin und München, Postfach 22 02 61, D-8000 München 22 (DE)**

(72) Erfinder: **Dietz, Kurt, Saarstrasse 18, D-8520 Erlangen (DE)**

(54) Röntgenröhre.

(57) Die Erfindung bezieht sich auf Röntgenröhren zur Herstellung von Körperschichtbildern mittels eines abtastend bewegten Röntgenstrahlenbündels, das auf einem ringförmigen Bereich eines Anodenkörpers erzeugt wird. Dazu liegen der Anode gittergesteuerte Kathoden in Abstand gegenüber und die Gitter werden nacheinander in einer der Abtastgeschwindigkeit entsprechenden Sequenz (Bildanzahl und Belichtungszeit) freigeschaltet. Zur Erhöhung der Belastbarkeit wird der ringförmige Bereich um eine Achse gedreht, auf der die Zentren des Bereichs und der Kathoden liegen. So kann die Geschwindigkeit unabhängig von der Herstellung der Bilder auf diejenige gebracht werden, die bei Drehanoden-Röntgenröhren benutzt ist und bei der eine optimale Verteilung der entstehenden Wärme erfolgt. Eine erfindungsgemäße Röntgenröhre ist universal anwendbar, weil auch einzelne Kathoden einschaltbar sind. Insbesondere ist sie für den Einsatz zur Herstellung von Röntgenschichtbildern nach dem Zonoskopieverfahren geeignet.

SIEMENS AKTIENGESELLSCHAFT        Unser Zeichen
Berlin und München                VPA 81 P 5018   E

Röntgenröhre

Die Erfindung betrifft eine Röntgenröhre nach dem Oberbegriff des Patentanspruchs 1. Solche Röhren sind z.B.
bekannt aus der DE-OS 26 47 167.

Bei der bekannten Röhre soll die Anode in einer Ausführungsform aus einem massiven Balken aus Wolfram bestehen.
Dieser kann kreisförmig zu einem Ring gebogen sein, dem
in Abstand voneinander mehrere Kathoden mit Sperrgittern
zugeordnet sind. Dabei ist aber die Belastbarkeit begrenzt.

Der Erfindung liegt die Aufgabe zugrunde, eine Röntgenröhre gemäß dem Oberbegriff des Patentanspruchs 1 anzugeben, deren Aufbau und Betrieb insbesondere hinsichtlich der Belastbarkeit verbessert ist. Diese Aufgabe
wird erfindungsgemäß durch die im kennzeichnenden Teil
des Anspruchs 1 angegebenen Maßnahmen gelöst.

Ausgehend von dem Nachteil, daß bei bekannten Vielkathodenröhren der vorliegenden Art die Belastbarkeit zur Erzeugung von Schichtbildern gering ist, haben Versuche,
die zu vorliegender Erfindung führten, gezeigt, daß eine Relativbewegung zwischen Kathode und Anode nicht in
der erwarteten Weise schon durch die für die Abtastung
die zur Erhöhung der Belastung ausreichende Erhöhung der
Belastbarkeit gewährleistet. Mit ihr wird noch keine
hinreichende Verteilung der bei der Abbremsung der Elektronen entstehenden Wärme erhalten. Eine genügende Verteilung wird erhalten, wenn die Anode zusätzlich in die
bei Drehanoden übliche Rotation um das Zentrum der An-

Kn 5 Kof / 16.03.1981

ordnung der Brennflecke versetzt wird. Die Rotation kann im Gegensatz zu der "Abtast-Rotation", die an die Bilderzeugung hinsichtlich Geschwindigkeit und Belichtungszeit gebunden ist, optimal an die Verteilung der Wärme angepaßt werden. Erst dann wird die an sich schon bei der kreisenden Abtastung erwartete Steigerung der Belastbarkeit der Anode möglich.

In einer wegen der Gittersteuerung günstigen Ausführungsform ist eine Röntgenröhre nach der Erfindung in einpoliger Metall-Glas-Konstruktion mit axialem Strahlenaustritt und geerdeten Kathoden ausgebildet. Dabei ist der Teil, der die Kathoden enthält, als eine Art topfförmiger Deckel für ein den Anodenteil enthaltendes gläsernes Unterteil ausgebildet. Die Kathoden sind seitlich der Anodenscheibe an der Innenwand des metallenen Röhrenkolbenteils angesetzt. Die Strahlen werden der Röhre in Richtung der Drehachse entnommen. Dazu ist der Boden des metallenen Deckelteils als Strahlenaustrittsfenster ausgestattet. Es kann aus einer röntgendurchlässigen Metallfolie bestehen, etwa aus einer ca. 0,15 mm dicken Folie aus Titan.

Bei einer Anode von 10 bis 20 cm Durchmesser können 10 bis 20 Kathoden in gleichmäßigen Abständen voneinander benutzt sein. Eine der Kathoden kann als eine Art Hauptkathode ausgebildet sein, die auch unabhängig von den anderen benutzbar ist. Damit sie den an sie gestellten Anforderungen gewachsen ist, kann sie in parallelen Schlitzen nebeneinander mehrere, getrennt voneinander einschaltbare Glühwendeln enthalten. Um für besondere Fälle auch höhere Röhrenspannungen anwenden zu können, ist es zweckmäßig, wenigstens die Hauptkathode gegen den metallischen Kathodenteil zu isolieren. Damit kann zwischen Anode und Hauptkathode eine höhere Spannung angewendet werden.

0062219

Aus der Vielzahl von Kathoden ist auch eine Einschaltung von nur solchen möglich, die in Stereoabstand voneinander stehen. Zusätzlich zu der einen Hauptkathode - oder einer anderen - kann noch eine zweite Kathode - in grösserem oder kleinerem Abstand davon - zuschaltbar ausgebildet sein, die dann wechselweise mit der erstgenannten zur Herstellung von Stereoaufnahmen und Stereo-Serienaufnahmen sowie Stereokino anwendbar ist. Insgesamt hat sich gezeigt, daß vorliegende Röhre wegen der gesteigerten Belastbarkeit zusätzlich zur Zonoskopie universell für die Anwendung bei allen bekannten Röntgenaufnahmeverfahren einsetzbar ist.

Einzelheiten und Vorteile der Erfindung sind nachfolgend anhand der in den Figuren dargestellten Ausführungsbeispiele weiter erläutert.

In der Figur 1 ist ein Querschnitt durch eine erfindungsgemäß mit 12 Kathoden ausgestattete Röhre gezeichnet,

in der Figur 2 eine Draufsicht auf eine entlang der Linie II-II aufgeschnittene Röhre nach Figur 1 und

in der Figur 3 ein schematisch dargestellter Ausschnitt aus einer Röhre mit topfförmiger Anode, der die Kathoden an der Innenseite der Seitenwand zugeordnet sind.

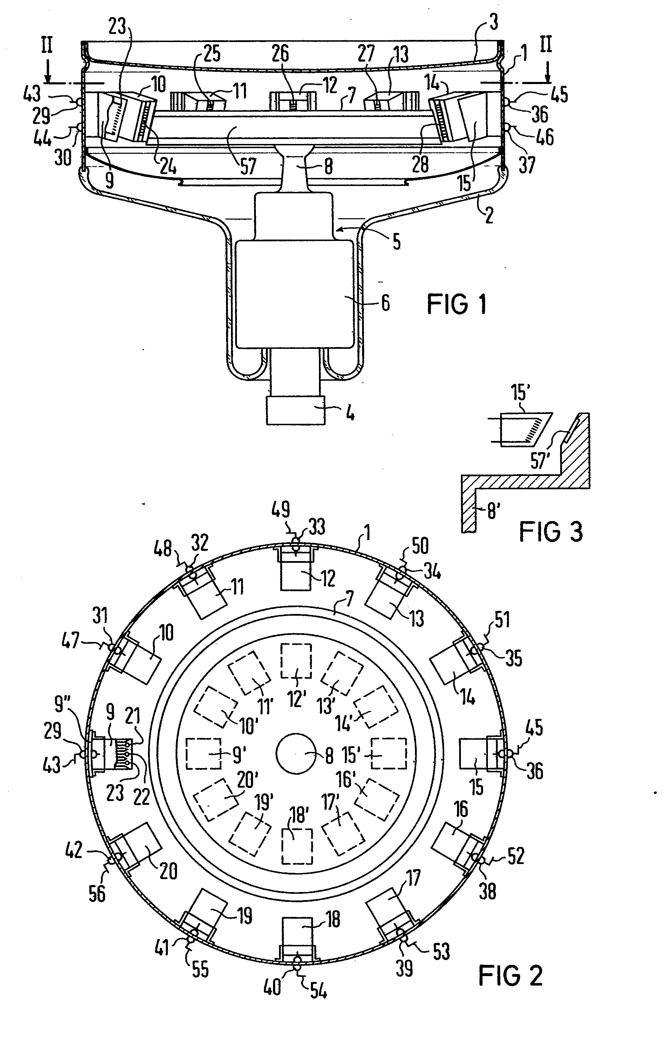

In der Figur 1 ist mit 1 ein Metallzylinder bezeichnet, der zusammen mit einem Teil 2 aus Glas den zylinderförmigen Teil eines Röhrenkolbens darstellt, dessen eine Stirnseite mit einem Strahlenaustrittsfenster 3 verschlossen ist, während die eine Verjüngung aufweisende

gegenüberliegende Stirnseite mit einem Anschlußstutzen 4 verschlossen ist. Der Stutzen 4 trägt eine Drehanodenanordnung 5, die in bekannter Weise aus einem Rotor 6 und einem Anodenkörper 7 besteht, die über eine Achse 8 miteinander fest verbunden sind.

Dem äußeren Umfang der Anode 7 sind Glühkathoden 9 bis 20 zugeordnet, indem sie an der Innenwand des Metallzylinders 1 angebracht sind. Davon ist die Kathode 9 über eine elektrische Isolierung 9'' am Metallzylinder 1 als Hauptkathode ausgebildet, indem sie drei Glühkathodenwendeln 21 bis 23 enthält, während im vorliegenden Beispiel alle übrigen nur eine Glühkathodenwendel enthalten, von denen die mit 24 bis 28 bezeichneten in der Figur 1 sichtbar sind. An sich können natürlich in allen Kathoden mehrere, z.B. zwei, Wendeln enthalten sein. Die Wendeln 23 bis 28 weisen über Isolierkörper 29 bis 42 durch die Wand 1 hindurchgeführte Anschlüsse 43 bis 56 auf. So ist es möglich, die Glühwendeln 23 bis 28 zur Abgabe von Elektronen zu bringen, die dann auf die Brennfleckbahn 57 der Drehanode 7 gelangen und Röntgenstrahlen auslösen, die bei der dargestellten Abschrägung durch das Fenster 3 aus der Röhre austreten können.

Die Halter der Wendeln 21 bis 28 dienen gleichzeitig als Schaltgitter, indem zwischen Glühwendel und Kathodenkopf eine negative Spannung angelegt wird, wenn aus der Kathode keine Elektronen austreten sollen.

Für die Herstellung von Stereoaufnahmen kann die Kathode 9 zusammen mit der Kathode 11 benutzt werden, weil diese den benötigten "Stereoabstand" hat.

Werden Kathoden, wie in Figur 2 gestrichelt angedeutet, mit 9' bis 20' auch immer, d.h. zwischen Achse 8 und Anodenkörper 7, angebracht, dann kann bei gleichem Fokus-Film-Abstand der Schichtwinkel verkleinert werden.

Man kommt dem idealen Schichtbild mit flächenhafter Verwischung näher, wenn man für eine Schicht zwei Verwischungskreise anwendet.

3 Figuren

8 Patentansprüche

Patentansprüche

1. Röntgenröhre zur Herstellung von Körperschichtbildern mittels eines abtastend bewegten Röntgenstrahlenbündels, das auf einem ringförmigen Bereich eines Anodenkörpers erzeugt wird, mittels gittergesteuerter, in Abstand voneinander dem Bereich gegenüberstehender Kathoden erzeugt wird, indem die Gitter nacheinander in einer der Abtastgeschwindigkeit entsprechenden Sequenz (Bildanzahl und Belichtungszeit) freigeschaltet werden, d a d u r c h   g e k e n n z e i c h n e t , daß der ringförmige Bereich um sein Zentrum gedreht wird, auf dem die Zentren des Bereichs und der Kathodenanordnung liegen, in einer Geschwindigkeit, die eine bei Drehanoden-Röntgenröhren auftretende Relativbewegung zwischen Kathoden und Elektronenaufprallfläche ergibt.

2. Röhre nach Anspruch 1,   d a d u r c h   g e - k e n n z e i c h n e t ,   daß der ringförmige Bereich an der Wand eines Topfes liegt, der um das Zentrum seines Bodens gedreht wird.

3. Röhre nach Anspruch 2,   d a d u r c h   g e - k e n n z e i c h n e t ,   daß sowohl auf der Außenseite als auch auf der Innenseite der Wand sich ein ringförmiger Bereich befindet und daß sowohl dem äußeren als auch dem inneren Bereich eine Kathodenanordnung zugeordnet ist.

4. Röhre nach Anspruch 3,   d a d u r c h   g e - k e n n z e i c h n e t ,   daß die Kathoden der beiden Anordnungen gegeneinander versetzt liegen.

5. Röhre nach Anspruch 2,   d a d u r c h   g e - k e n n z e i c h n e t ,   daß eine Kathode (die Hauptkathode) von den anderen Kathoden elektrisch isoliert angebracht ist.

0062219

- 7 -VPA 81 P 5018  E

6. Röhre nach Anspruch 1, d a d u r c h. g e -
k e n n z e i c h n e t , daß zwei Kathoden voneinander getrennt von den anderen Kathoden einschaltbar sind.

7. Röhre nach Anspruch 3, d a d u r c h g e -
k e n n z e i c h n e t , daß der Abstand der beiden Kathoden dem zur Aufnahme von Stereobildern ausreichenden Abstand entspricht.

8. Röhre nach Anspruch 1, d a d u r c h g e -
k e n n z e i c h n e t , daß auch die Kathodenanordnung drehbar ist, in einer Geschwindigkeit, die
der Abtastgeschwindigkeit geteilt durch die Zahl der
Kathoden wenigstens nahekommt.

0062219

FIG 1

FIG 3

FIG 2